# EUROPÄISCHE PATENTSCHRIFT

(11) **EP 1 720 611 B1**
(45) Veröffentlichungstag und Bekanntmachung des Hinweises auf die Patenterteilung: **19.05.2010**
(21) Anmeldenummer: 05707434.6
(22) Anmeldetag: 16.02.2005
(51) Int. Cl.: A61P 35/00, G01N 33/574

(54) **DIAGNOSTISCHE MARKER FÜR KREBS**
DIAGNOSTIC MARKER FOR CANCER
MARQUEUR DIAGNOSTIQUE POUR LE CANCER

(30) Priorität: 16.02.2004 DE 102004008449; 29.07.2004 DE 102004038076
(43) Veröffentlichungstag der Anmeldung: 15.11.2006
(73) Patentinhaber: ProteoSys AG, 55129 Mainz (DE)
(72) Erfinder: CAHILL, Michael, 55296 Lörzweiler (DE); KLOCKER, Helmut, A-6401 Inzing (AT); ROGATSCH, Hermann, A-9020 Klagenfurt (AT)
(74) Vertreter: Weiss, Wolfgang
(86) Internationale Anmeldenummer: PCT/EP2005/001567
(87) Internationale Veröffentlichungsnummer: WO 2005/078124

(56) Entgegenhaltungen:
- US-A1- 2002 119 463
- US-A1- 2003 108 963
- US-A1- 2003 180 738
- US-A1- 2003 185 808
- US-B1- 6 476 207
- HOFMANN E A: "Interactions of benzodiazepine drivatives with annexins" JOURNAL OF BIOLOGICAL CHEMISTRY, AMERICAN SOCIETY OF BIOLOGICAL CHEMISTS, BALTIMORE, MD, US, vol. 273, no. 5, 30 January 1998 (1998-01-30), pages 2885-2894, XP002098631 ISSN: 0021-9258 cited in the application
- VAARALA M H ET AL: "Differentially expressed genes in two LNCaP prostate cancer cell lines REFLECTING CHANGES DURING PROSTATE CANCER PROGRESSION" LABORATORY INVESTIGATION, UNITED STATES AND CANADIAN ACADEMY OF PATHOLOGY, BALTIMORE,, US, vol. 80, no. 8, August 2000 (2000-08), pages 1259-1268, XP002225395 ISSN: 0023-6837
- GRANER EDGARD ET AL: "The isopeptidase USP2a regulates the stability of fatty acid synthase in prostate cancer" CANCER CELL, vol. 5, no. 3, March 2004 (2004-03), pages 253-261, XP002340626 ISSN: 1535-6108
- KÖLLERMANN ET AL.: "Expression and Prognostic Relevance of Annexin A3 in Prostate Cancer" EUROPEAN UROLOGY, 2008, Seiten 1-10,
- WOJCIECH ET AL.: "Differential radioactive quantification of protein abundance ratios between benign and malignant prostate tissues: Cancer association of annexin A3" PROTEOMICS, Bd. 7, Nr. 2, 2007, Seiten 313-322,
- SHEKOUH A R ET AL.: "Application of laser capture microdissection combined with two-dimensional electrophoresis for the discovery of differentially regulated proteins in pancreatic ductal adenocarcinoma" PROTEOMICS, Bd. 3, Nr. 10, Oktober 2003 (2003-10), Seiten 1988-2001, Germany
- GASSER O ET AL.: "Characterisation and properties of ectosomes released by human polymorphonuclear neutrophils." EXPERIMENTAL CELL RESEARCH, Bd. 285, Nr. 2, 1. Mai 2003 (2003-05-01), Seiten 243-257, USA

## Beschreibung

Die vorliegende Erfindung betrifft die Verwendung

des Proteins Annexin A3 als diagnostischer Marker für Prostatakrebs, dadurch gekennzeichnet, dass es sich bei dem Prostatakrebs um bestimmte Unterarten von Prostatakrebs handelt.

Krebserkrankungen sind im allgemeinen durch die Entstehung von einem oder mehreren Tumoren gekennzeichnet. Unter Tumor wird eine Geschwulst bzw. die örtlich umschriebene Zunahme des Gewebevotumens verstanden. Im weiteren Sinne kann jede lokalisierte Anschwellung, z. B. durch ein Ödem, einer akuten und chronischen Entzündung, aneurysmatische Erweiterung, einer entzündlich bedingten Organschwellung (z. B. als sogenannter Milztumor) verstanden werden. Im engeren Sinne werden unter Tumor gewebliche Neubildungen (Gewächs, Plastom, Neoplasie) in Form eines spontanen, verschiedengradig enthemmten, autonomen und irreversiblen Überschußwachstums von körpereigenem Gewebe, das in der Regel mit unterschiedlich ausgeprägtem Verlust spezifischer Zell- und Gewebefunktion verbunden ist, verstanden.

Die Tumore werden zur besseren Klassifikation unterteilt in:
I. nach ihrem biologischen Verhalten:
   1. benigne (gutartige) Tumore mit differenzierten Zellen und langsamen, lokal verdrängendem Wachstum.
   2. maligne (bösartige) Tumore mit Zellkernpolymorphie, Zellatypie, Anaplasie und infiltrierendem, meist raschem, destruierendem Wachstum und Metastasierung.
   3. semimaligne Tumore mit den histologischen Kennzeichen maligner Tumore und lokal infiltrierendem Wachstum, jedoch in der Regel ohne eine Metastasierung.
II. histogenetische Systematik:
   Hierbei werden die Tumore klassifiziert anhand des Gewebes, aus dem sie entwicklungsgeschichtlich hervorgegangen sind. Es gibt:
   1. epitheliale Tumore, die aus Ektoderm und Entoderm hervorgegangen sind:
      a) benigne Tumore wie z. B. Adenom, Papillom und Polypen.
      b) maligne Tumore wie z. B. Karzinom.
   2. mesenchymale Tumore, hervorgegangen aus dem Mesoderm:
      a) benigne Tumore wie z. B. Lipom, Fibrom, Osteom, Myom, Leiomyom, Rhabdomyom, Chondrom,
      b) maligne Tumore wie z. B. die Sarkome.
   3. embryonale Tumore sind aus undifferenziertem Gewebe hervorgegangen. Hierzu zählen z. B. Nephroblastome, Neuroblastome, Medulloblastome, Retinoblastome sowie embryonale Rhabdomyosarkome und Teratome.
III. Klassifikation nach klinischen und pathologischen Befunden: Unter anderem gelten hier die TNM-Klassifikation, Grading, Lauren-Klassifikation, Dukes-Klassifikation, Kieler-Klassifikation, Rappaport-Klassifikation etc.

Schon diese kurze Übersicht der Tumoreinteilung zeigt, welche Vielfalt (und zum Teil Gegensätzlichkeit) innerhalb der verschiedenen Tumorarten besteht. So ist z. B. nicht nur zwischen benignen und malignen Tumoren zu unterscheiden, sondern auch zwischen Mortalität bzw. Letalität der einzelnen Tumore und die Wahrscheinlichkeit, daß sich ein benigner Tumor zu einem malignen Tumor weiterentwickelt.

Einzelne Tumore wie z. B. die Mamakarzinome (Brustkrebs), der häufigste maligne Tumor der Frau, treten gehäuft vor allem zwischen dem 45. und 70. Lebensjahr auf. Frühsymptome sind verdächtige Tastbefunde, die in der Regel infolge der Krebsfrüherkennungsuntersuchungen sowie bei regelmäßiger Selbstuntersuchung der Brust entdeckt werden. Abhängig von Tumorstadium und Differenzierungsgrad des Tumors kann dabei die Prognose von durchaus positiv bis sehr schlecht ausfallen. Infolge der frühen lymphogenen und hämatogenen Metastasierung bei Mammakarzinomen kommt es auf eine rasche Diagnostizierung des Tumors an, um frühestmöglich mit der Therapie einsetzen zu können.

Prostatakarzinome (Karzinom der Prostata) ist demgegenüber der häufigste maligne Tumor des Mannes, der vor allem zwischen dem 50. und 70. Lebensjahr auftritt. In der Mehrzahl handelt es sich dabei um Adenokarzinome. Dieser maligne Tumor breitet sich durch infiltrierendes Wachstum zunächst innerhalb der Prostata aus, später erfolgt eine Infiltration von Bläschendrüsen und Beckenbindegewebe, relativ selten auch von Rektum, Harnblase oder Urethra. Die Metastasierung erfolgt lymphogen und/oder hämatogen. Die Therapie erfolgt abhängig vom histologischen Differenzierungsgrad und klinischen Stadium in der Regel durch radikale Prostatektomie mit regionaler Lymphknotenausräumung, im fortgeschrittenen Stadium Entzug der männlichen Sexualhormone. Auch hierbei ist die Prognose abhängig vom Stadium des Karzinoms. Während in einem sehr frühen Stadium nach einer radikalen Prostatektomie in ca. 90 % der Fälle eine Heilung eintritt, ist bei fortgeschrittenem Stadium eher mit einer pessimistischen Prognose zu rechnen.

Prostatakarzinome sind von Prostatahyperplasie in der Diagnose zu unterscheiden. Bei der Prostatahyperplasie handelt es sich um einen benignen, also gutartigen Tumor. Dabei vergrößert sich die Prostata durch numerische Zunahme der Zellen und Drüsen des Stromas. Die Prostatahyperplasie ist die häufigste Ursache von Blasenentleerungsstörungen bei Männern. Klinisch beginnt sie vor allem zwischen dem 40. und 50. Lebensjahr. Der Verlauf ist langsam und schubweise. Das Auftreten von Beschwerden erfolgt dabei meistens erst nach Jahren mit allmählicher Abschwächung des Harnstrahls und verzögertem Miktionsbeginn. Hierbei kann als Therapie bzw. Linderung der Symptomatik die Verabreichung von Phytotherapeutika in Betracht gezogen werden.

Da generell eine frühe Erkennung, d. h. Diagnostizierung, der Tumore für den raschen Therapiebeginn wichtig ist und auch die Prognose um so besser ist, je früher der Tumor erkannt wird, sind eine Reihe von sogenannten Tumormarkern im klinischen Einsatz. Als Tumormarker werden dabei allgemein Substanzen und zelluläre Veränderungen bezeichnet, deren qualitative oder quantitative Analyse eine Aussage über Vorliegen, Verlauf oder Prognose von (bösartigen) Erkrankungen ermöglichen kann. Eingeteilt werden Tumormarker in:
1. Zelluläre Tumormarker:
   Darunter fallen unter anderem zellmembranständige Tumorantigene, Rezeptoren (z. B. Hormonrezeptoren, Rezeptoren für wachstumsfördernde Substanzen bei Leukämie) und Zellmarker, die auf eine vermehrte Expression von Onkogenen und ein monoklonales Zellwachstum hindeuten, sowie molekulargenetische zelluläre Veränderungen, vor allem Chromosomenaberrationen.
2. Humorale Tumormarker:
   Diese sind gegenüber physiologischen Bedingungen in Körperproben, insbesondere in Serum, Urin und anderen Körperflüssigkeiten in erhöhten Konzentrationen nachweisbare (meist physiologisch vorkommende) Substanzen, die vom Tumorgewebe synthetisiert und/oder sezerniert, durch Tumorzerfall freigesetzt oder als Reaktion des Organismus auf einen Tumor gebildet werden. Die physiologische Bedeutung von Tumormarkern ist nur unzureichend bekannt. Im menschlichen Organismus wirken sie in der Regel nicht immunogen. Die klinische (diagnostische) Bedeutung ist abhängig von ihrer Spezifität und Sensitivität. Die humoralen Tumormarker werden in zwei Gruppen unterteilt. In der ersten Gruppe werden die humoralen Tumormarker zusammengefaßt, die vom Tumor selbst produziert werden. Darunter fallen z. B. tumorassoziierte Antigene, bestimmte Hormone (z. B. Gastrin, Cortisol etc.), Enzyme (z. B. neuronspezifische Enolase (NSE)), sowie Proteine (z. B. Bence-Jones-Protein). In der zweiten Gruppe sind die Tumormarker enthalten, die vom Tumor zwar induziert, aber nicht selbst produziert werden. Wichtige humorale Tumormarker dieser Gruppe sind z. B. alkalische Phosphatase (AP), LDH, Neopterin etc.

Kürzlich ist eine Liste von Proteinen, die in zwei repräsentativen Zelllinien des Medulloblastoms, des häufigsten Gehirntumors bei Kindern, nachgewiesen werden konnten und möglicherweise als Tumormarker verwendet werden können, veröffentlicht worden [A. Peyrl et al., 2003, Proteomics, 3, 1781-1800]. Aus der US 6,645,465 ist bekannt, dass die zu den Ca²⁺-bindenden Proteine gehörenden Annexine A1 und A2 als Tumormarker für Lungen-, Brust- und Speiseröhrenkrebs verwendet und durch Detektion von gegen sie gerichtete Auto-Antikörper nachgewiesen werden können. In Tierversuchen konnte gezeigt werden, daß der Einsatz von radioaktiv markierten Antikörpern gegen Annexin A1 zu einem Verlust an Tumormasse führt, was vermutlich auf den Zelltod der Tumorzellen zurückzuführen ist [P. Oh, Y. Li; J. Yu, E. Durr, K. M. Krasinska, L. A. Carver, J. E. Testa, J. E. Schnitzer, 2004, Nature, 429, 629-35.].
Weiterhin ist neulich eine differentielle Abundanzanalyse in malignen und nicht-malignen (benignen) Pankreasepithelzellen durchgeführt worden, wobei Annexin A3 als identifiziertes Protein in diesem Zusammenhang aufgeführt ist [A. R. Shekouh et al., 2003, Proteomics, 3, 1988-2001]. Ferner ist die Abundanz von Proteinen in malignem und nicht malignem Prostatagewebe untersucht worden. Bezüglich der in diesem Zusammenhang identifizierten Proteine werden allerdings größtenteils keine näheren Angaben zu einer möglichen Über- oder Unterexpression der aufgeführten Proteine im karzinomatösen Gewebe im Vergleich zum gesunden Gewebe gemacht [A. A. Alaiya et al., 2001, Cell. Mol. Life Sci., 58, 307-311]. US2003/0108963 beschreibt die Verwendung von Annexin A3 als allgemeinenProstatakrebsmarker.

Die bisher gemachten Aussagen zeigen, wie wichtig selektive und sensitive Nachweisverfahren für Tumore sind. Weiterhin besteht ein großer Bedarf an neuen Targets bei der Tumor- bzw. Krebstherapie.

Dementsprechend stellt sich die Erfindung die Aufgabe, neue diagnostische Marker für Krebserkrankungen sowie neue Angriffspunkte und Wirkstoffe für die Krebstherapie bereitzustellen.

Diese Aufgabe wird durch die Gegenstände der unabhängigen Ansprüche gelöst. Bevorzugte Ausführungsformen sind in den abhängigen Ansprüchen genannt. Der Wortlaut sämtlicher Ansprüche wird hiermit durch Bezugnahme zum Inhalt der Beschreibung gemacht.

Es konnten durch intensive vergleichende Analysen zwischen bösartig entartetem Gewebe (Krebsgewebe) und nicht entartetem Gewebe verschiedene Proteine identifiziert werden, die sich bei diesen Gewebearten in ihrer Häufigkeit bzw. Konzentration (Abundanz) signifikant unterscheiden. Die charakteristische Abundanz eines bestimmtes Proteins im Vergleich mit Kontrollen stellt daher ein wichtiges Indiz für das Vorhandensein von entartetem Zellwachstum, also Krebsgewebe, dar. Erfindungsgemäß werden diese Proteine als diagnostische Marker für Krebs verwendet.

Zur Identifizierung dieser Proteine wurden Proben aus Krebsgewebe (Prostatakrebs) und gesundem Prostatagewebe aufbereitet und die beiden Proben jeweils mit verschiedenen radioaktiven Isotopen markiert. Die Proben wurden zusammengemischt und gemeinsam auf einem zweidimensionalen Polyacrylamidgel elektrophoretisch aufgetrennt. Die Signale jedes Isotops wurden getrennt detektiert und die entsprechenden Proteinspots weiter analysiert. Mit dieser Vorgehensweise konnten verschiedene Proteine identifiziert werden, die sich in ihrer Häufigkeit im Krebsgewebe deutlich von der Häufigkeit im gesunden Gewebe unterscheiden. Die verschiedenen Proteine sind hierbei zum Teil in Krebsgewebe deutlich häufiger vorhanden bzw. abundant, also heraufreguliert, und zum Teil deutlich weniger vorhanden bzw. abundant, also herabreguliert.

Die Erfindung umfaßt die Verwendung des Proteins Annexin A3 als diagnostischen Marker für Prostatakrebs. Die Erfinder konnten zeigen, dass dieses Protein in Krebsgewebe durchschnittlich 2,4-fach, in bestimmten Patientengruppen sogar mehr als 5-fach heraufreguliert ist. In einer besonders bevorzugten Ausführungsform kann Annexin A3 folglich als diagnostischer Marker für bestimmte Unterarten (Patientengruppen) von Prostatakrebs verwendet werden. Daher wird vorzugsweise eine Heraufregulation dieses Proteins im Vergleich mit Kontrollen als charakteristisches Merkmal für Krebsgewebe untersucht. Die Annexine sind eine Familie von strukturell verwandten Proteinen, die Phospholipide in Abhängigkeit von Kalzium binden und Kalziumporen bilden können. Die genaue Rolle der Annexine ist bislang jedoch unklar.

Es wird vermutet, daß die Annexine sowohl an intrazellulären als auch an extrazellulären Vorgängen, beispielsweise Membran-Trafficking, Zellbeweglichkeit, Ca²⁺-Influx und Signaltransduktion, beteiligt sind. Allerdings ist unbekannt, auf welche Weise Annexine sekretiert werden. Beispielsweise besitzen sie keine klassischen Leadersequenzen für eine Translation in das Lumen des endoplasmatischen Retikulums. Da Annexine jedoch zum Teil in kleinen sekretierten Membran-Vesikeln mit einem Durchmesser von 30-100 nm, den sogenannten Exosomen, gefunden wurden, wird vermutet, daß Annexine durch eine Lyse dieser Exosomen das Äußere der Zelle erreichen können. Die Lyse dieser Vesikel kann zu einer veränderten Antigen-Präsentation in Tumoren führen. Generell spielen Exosomen bei der Antigen-Präsentation im Immunsystem eine Rolle und sind dabei in dem MHC-Klasse 1/T-Zell-System involviert.

Interessanterweise sind Annexine in den Vorgang der Knochenmineralisation involviert (Wang, W., Xu, J., Kirsch, T. 2003, Annexin-mediated Ca2+ influx regulates growth plate chondrocyte maturation and apoptosis. J. Biol. Chem. 2003, 278: 3762-9). Dies ist besonders bemerkenswert, da Prostatakrebsmetastasen im Vergleich mit anderen Krebsarten eine ungewöhnlich hohe Frequenz von osteoblastischen Knochenläsionen verursachen. Die meisten Krebsmetastasen sind durch eine osteolytische Aktivität, d. h. also durch einen Knochenabbau, charakterisiert, Prostatakrebsmetastasen hingegen zeigen sowohl osteoklastische, also abbauende, als auch osteoblastische, also knochenaufbauende, Aktivität. Hierfür werden zunächst normale Knochenkristalle abgebaut und dann als ungeordnete Knochenablagerungen wieder aufgebaut. Obwohl dieser Mechanismus nur wenig verstanden ist, spielen hier vor allem physiologische Mineralisierungsprozesse eine Rolle. Eine Mineralisierung wird durch kleine Vesikel, sogenannte Matrixvesikel, initiiert, welche von den Plasmamembranen mineralisierende Skelettzellen (Osteoblasten) freigesetzt werden. Die erste mineralische Phase (Kalziumphosphatkristalle) formt sich innerhalb der Matrixvesikel. Da diese Vesikel von Membranen umschlossen sind, werden Kanalproteine benötigt, damit die Mineralien in die Vesikel eindringen können. Wichtige Komponenten dieser Vesikel sind die Annexin-Proteine, z.B. Annexin A2, A5 und A6 sowie Collagen-Typ II und X an der äußeren Oberfläche der Vesikel, welche über Bindung an Annexin A5 an die äußere Vesikeloberfläche angelagert werden. Die Annexine bilden Kanäle in den Membranen der Matrixvesikel, durch welche Ca²⁺ in die Vesikel eindringt. Collagen, welches an Annexin A5 gebunden ist, verstärkt diese Kanalaktivitäten und vermittelt mit anderen Annexinen den schnellen Ca²⁺-Influx und die Bildung der ersten kristallinen Phase innerhalb der Vesikel. Dies führt zur Initiierung der Mineralisation. Wenn die intrazellulären Kristalle eine bestimmte Größe erreicht haben, zerstören sie die Membran und lysieren die Vesikel. Die Kristalle wachsen weiter (Wachstumsphase der Mineralisation) und tragen zum Knochenaufbau bei. Gemäß den Ergebnissen der Erfinder ist diese Rolle der Annexine bei der ungewöhnlichen Knochenmineralisation durch Prostatakrebsmetastasen vermutlich mit der Hochregulation von Annexin A3 in Prostatakrebsgewebe verknüpft. In diesem Zusammenhang ist auch die anorganische Pyrophosphatase zu sehen, die anorganisches Phosphat freisetzt, und welche gemäß den Ergebnissen der Erfinder in Krebs, insbesondere in Prostatakrebs, heraufreguliert wird. Aus der Hochregulation von Annexin A3 in Prostatakrebszellen ist zu schließen, daß Annexin A3 eine biologische Rolle in Exosomen der Prostatakrebszellen spielt. Hierfür kommt vor allem ein Zusammenhang mit lonenkanälen in Frage. In einer bevorzugten Verwendung des Proteins Annexin A3 wird daher die Aktivität des Proteins in Exosomen beeinflußt. Vorzugsweise führt dies zu einer geänderten Immunüberwachung und metastasischen Eigenschaften der Tumorzeilen. Da extrazelluläres Annexin A3 eine höhere Konzentration in der Nähe der Tumorzellen aufweist, kann ein Affinitätsreagens, insbesondere ein therapeutischer Antikörper, welcher eine hohe Affinität zu Annexin A3 besitzt, genutzt werden, um Wirkstoffe wie beispielsweise Toxine oder radioaktive Dosen, in die Nähe des Tumors zu befördern. Solch ein Wirkstoff sollte vorzugsweise nicht die Zellmembran wirksam durchqueren, damit vorteilhafterweise gesunde Zellen, welche lediglich intrazelluläres Annexin A3 exprimieren, hiervon nicht beeinflußt werden. Interessanterweise wurden Matrixvesikel ebenfalls im Zusammenhang mit osteoarthritischem Knorpel und arteriosklerotischen Läsionen beobachtet.

Die Freisetzung zytoplasmatischer Proteine in das extrazelluläre Medium, welche nach Lyse der Exosomen erfolgt, kann eine entzündliche Antwort induzieren, die ähnlich der bei einer Zellnekrose ist. Es ist bekannt, daß eine Entzündung die adaptive T-Zellen-vermittefte Immunantwort verringern kann, was bekanntermaßen viele Krebszellen auszeichnet. Ferner kann die Anwesenheit von Annexinen im extrazellulären Raum diesen Verlauf ebenso beeinflussen (A. Bondanza et al., 2004, J. Exp. Med., 200, 1157-65). Deshalb könnte eine Impfung gegen Krebs durch Verstehen und Beeinflussung dieses Systems bestimmt werden.

In einer besonders bevorzugten Verwendung des Proteins Annexin A3 wird eine Heraufregulation dieses Proteins in Kombination mit einer Herabregulation von Annexin A1, Annexin A2 und/oder Annexin A5 untersucht. Dies geschieht vorzugsweise im Vergleich mit Kontrollen. Es wurde bereits gezeigt, daß Annexin A1, A2 und Annexin A5 in Krebsgewebe und insbesondere in Prostatakrebsgewebe herunterreguliert ist. Von daher ist eine Analyse der Heraufregulation von Annexin A3 in Kombination mit der Herunterregulation von einem oder mehreren dieser weiteren Annexine für eine Diagnose besonders aufschlußreich. Aufgrund der vorliegenden Ergebnisse könnte Annexin A3 andere Annexine während der Prostata-Karzinogenese ersetzen und daher ein Ersatzmarker oder ein Ersatz-Target für die Prostatakrebs-Behandlung sein.

Erfindungsgemäß konnte gezeigt werden, daß verschiedenen Proteine in charakteristischer Weise in Krebsgewebe im Vergleich mit gesundem Gewebe herunter- bzw. heraufreguliert sind. Einzelheiten hierzu ergeben sich aus der folgenden Tabelle 1. Hierin wird die Identifizierung und die Quantifizierung der verschiedenen Proteine in gutartigem und bösartigem Gewebe zusammengefaßt. Die Auswahl der Proteine basiert auf einer statistisch signifikanten differentiellen relativen Abundanz der Proteine in gutartigem (benigne Fraktion) und bösartigem (Krebsfraktion) Gewebe. Die *Accession Number* entspricht der jeweiligen Nummer aus der NCBI-Datenbank. Das theoretische Molekulargewicht (MW) ist das Molekulargewicht, das gemäß den Datenbanksequenzen errechnet wurde. Unter "scores" sind die mit Hilfe der MASCOT-Technik ermittelten Treffer zu verstehen. Die Angabe zum PMF-Score bezieht sich auf den Mowse-Score, der vom MASCOT-Server verwendet wird, wobei im allgemeinen ein PMF-Score, der höher als 65 ist, eine signifikante Identifizierung repräsentiert. Die letzten zwei Spalten fassen die Quantifizierung der Proteinspot-Intensitäten zusammen, die für die gutartigen und bösartigen Gewebefraktionen gefunden wurden.

**Tabelle 1:**

| **No** | **AccNo** | **Beschreibung** | **theor MW** | **PMF Score** | **benigne Fraktion** | **Krebs fraktion** |
|---|---|---|---|---|---|---|
| 1 | gi\|1732411 | isopeptidase T [Homo sapiens] | 94104 | 115 | 83.6 | 16.4 |
| 2 | gi\|576259 | Chain A; Serum Amyloid P Component (Sap) | 23598 | 106 | 73.1 | 26.9 |
| 3 | gi\|494781 | Fatty Acid Binding Protein (Holo Form, Human Muscle) (M-Fabp) | 14775 | 87 | 71.6 | 28.4 |
| 4 | gi\|4504981 | beta-galauosidase binding lectin precursor; Lectin; galactose-binding; soluble; 1; galectin [Homo sapiens] | 15769 | 177 | 66.2 | 33.8 |
| 5 | gi\|225159 | microseminoprotein beta | 12238 | 92 | 63.9 | 36.1 |
| 6 | | n.i. | | | 60.6 | 39.4 |
| 7 | gi\|662841 | heat shock protein 27 [Homo sapiens] | 22667 | 182 | 60.2 | 39.8 |
| 8 | gi\|4507949 | tyrosine 3-monooxygenase/tryptophan 5-monooxygenase activation protein, beta polypeptide; 14-3-3 pr | 27946 | 160 | 41.2 | 58.8 |
| 9 | gi\|4507953 | tyrosine 3/tryptophan 5 -monooxygenase activation protein, zeta polypeptide; protein kinase C inhib | 27810 | 160 | 41.1 | 58.9 |
| 10 | gi\|2073569 | nuclear chloride ion channel protein [Homo sapiens] | 27249 | 139 | 40.1 | 59.9 |
| 11 | | n.i. | | | 39.5 | 60.5 |
| 12 | | n.i. (Annexin A3) | 36524 | 160 | 37.4 | 62.6 |
| 13 | gi\|5803227 | tyrosine 3/tryptophan 5 -monooxygenase activation protein, theta polypeptide, 14-3-3 protein tau | 28032 | 130 | 35.6 | 64.4 |
| 14 | gi\|13129150 | heat shock 90kDa protein 1, alpha, heat shock 90kD protein 1, alpha [Homo sapiens] | 85006 | 147 | 32.6 | 67.4 |
| | gi\|20149594 | heat shock 90kDa protein 1, beta, heat shock 90kD protein 1. beta, Heat-shock 90kD protein-1, beta | 83554 | 164 | | |
| 15 | gi\|20070125 | prolyl 4-hydroxylase, beta subunit, v-erb-a avian erythroblastic Leukaemia viral oncogene homolog 2 | 57480 | 235 | 31.2 | 68.8 |
| 16 | gi\|4557581 | (NM_001444) fatty acid binding protein 5 (psoriasis-associated); E-FABP [Homo sapiens] | 15497 | 94 | 27.9 | 72.1 |
| 17 | gi\|2707570 | mitochondrial short-chain enoyl-coenzyme A hydratase 1 precursor [Homo sapiens] | 31807 | 101 | 26.2 | 73.8 |
| 18 | gi\|16307090 | Similar to nucleophosmin (nucleolar phosphoprotein B23, numatrin) [Homo sapiens] | 29617 | 77 | 21.9 | 78.1 |
| 19 | gi\|768772 | HES1 protein, homolog to E. coli and zebrafish ES1 protein, anti-sigma cross-reacting protein homolog 1 alpha precursor, KNP-la, GT335. similar to E. coli SCRP27A and to zebrafish ES1 [Homo sapiens] | 29215 | 95 | <20 | >80 |
| 20 | gi\|4506181 | proteasome alpha 2 subunit; proteasome subunit HC3; proteasome component C3: macropain subunit C3; multicatalytic endopeptidase complex subunit C3 [Homo sapiens] | 26236 | 105 | 32.6 | 67.4 |
| 21 | gi\|4502171 | adenine phophoribosyltransferase; AMP pyrophosphorylase; AMP diphosphorylase; transphosphoribosidase | 20127 | 134 | 33 | 67 |
| 22 | gi\|11056044 | inorganic pyrophosphatase [Homo sapiens] | | | 38.6 | 61.4 |

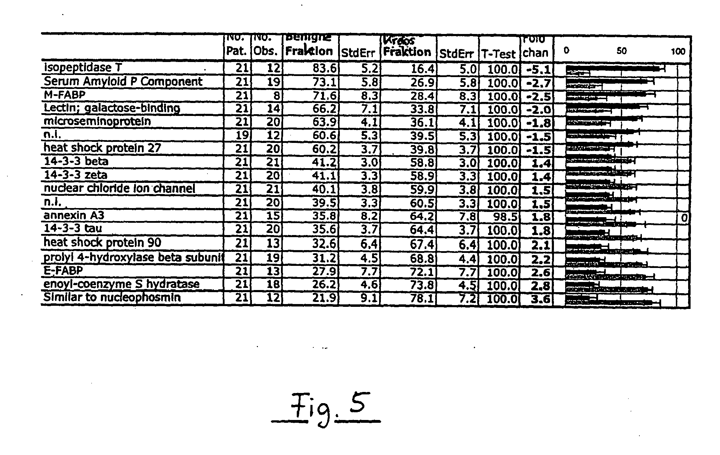

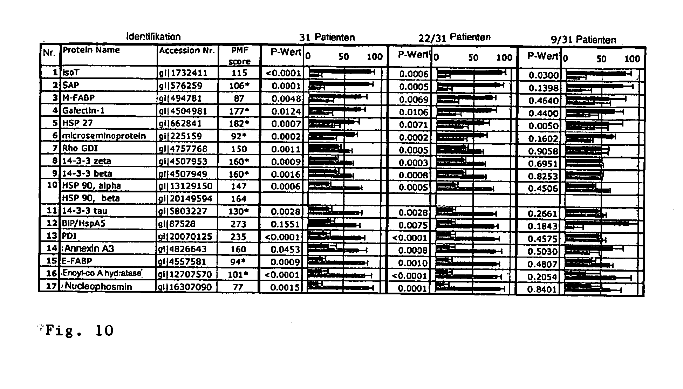

In diesem Zusammenhang wird weiterhin auf die Fig. 5 und 10 verwiesen, in welchen in tabellarischer Form die Ergebnisse der Proteinspots mit signifikanter differentieller durchschnittlicher Abundanz bei 21 Patienten bzw. 31 Patienten zusammen mit verschiedenen statistischen Werten dargestellt sind.

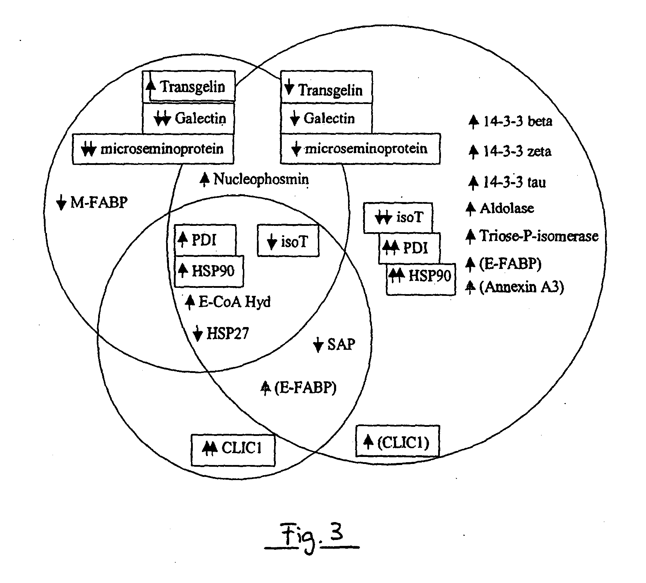

Im folgenden wird eine Zusammenstellung der englischsprachigen Synonyme für diese verschiedenen Proteine aufgelistet. Hierbei entspricht die vorangestellte Nummer der Numerierung in der Tabelle 1. An erster Stelle steht jeweils die in der übrigen Beschreibung gewählte deutsche Bezeichnung dieser Proteine.
1. gi|1732411: **Ubiquitin-Isopeptidase T;** Isopeptidase T (isoT); ubiquitin specific protease 5; Ubiquitin carboxyl-terminal hydrolase 5; Ubiquitin thiolesterase 5; Ubiquitin-specific processing protease 5; Deubiquitinating enzyme 5 ; de-ubiquitinase.
2. gi|576259: **Serum-Amyloid P-Komponente;** Chain A; Serum Amyloid P Component (SAP).
3. gi|494781: **Fettsäure-bindendes Protein 3 (FABP-3);** Mammaryderived growth inhibitor (MDGI); fatty acid binding protein 3 (FABP-3); Heart-Type Fatty Acid Binding Protein (H-FABP); Muscle-Type Fatty Acid Binding Protein (M-Fabp).
4. gi|4504981: **Galektin;** galectin-1; 14 kDa beta-galactoside-binding lectin; beta galactoside soluble lectin; Beta-galactoside-binding lectin L-14-1; Galaptin; soluble galactoside-binding lectin; S-Lac lectin 1.
5. gi|225159. **Mikroseminoprotein beta;** beta-microseminoprotein; microseminoprotein beta; Immunoglobulin binding factor (IGBF); PN44; Prostate secreted seminal plasma protein; Prostate secretory protein of 94 amino acids (PSP-94); Seminal plasma beta-inhibin; seminal plasma protein.
6. nicht identifiziert
7. gi| 662841: **Hitzeschockprotein 27 (HSP27);** heat shock protein 27; 27kDa heat shock protein 1 (HSP-27); Stress-responsive protein 27 (SRP27); Estrogen-regulated 24 kDa protein; 28 kDa heat shock protein.
8. gi|4507949: **14-3-3 Protein beta;** 14-3-3 protein beta (14-3-3 beta); 14-3-3 protein alpha (14-3-3 alpha); Protein kinase C inhibitor protein-1; PKC inhibitor protein-1 (KCIP-1: auch 14-3-3 zeta); RNH-1.
9. gi|4507953: **14-3-3 Protein zeta;** 14-3-3 zeta; 14-3-3 delta; KCIP-1 (auch 14-3-3 beta); YWHAZ; mitochondrial import stimulation factor S1 (MSF S1); Factor activating exoenzyme S; tryptophan monooxygenase activation protein zeta; tyrosine monooxygenase activation protein zeta.
10. gi|2073569: **nukleäres Chloridionenkanal-Protein;** chloride intracellular channel 1 (CLIC-1); nuclear chloride ion channel protein (p64CLCP); nuclear chloride channel; chloride channel ABP; Nuclear chloride ion channel 27 (NCC27); RNCC protein; Nuclear chloride ion channel 27 (NCC27).
11.: nicht identifiziert
12.: (Annexin A3, siehe 23.)
13. gi| 5803227: **14-3-3 Protein tau;** 14-3-3 tau; 14-3-3 theta; S15076 protein kinase regulator 14-3-3; HS1; tryptophan 5 -monooxygenase activation protein; tyrosine 3 -monooxygenase activation protein.
14. gi|13129150: **Hitzeschockprotein 90 (HSP90);** Heat shock protein 90 (HSP-90); Heat shock protein HSP 90-alpha; heat shock protein 90-alpha; 90 kDa heat-shock protein; heat shock protein 86 (HSP 86); Hspca; heat shock 90kDa protein 1; heat shock protein 1; Tumor specific transplantation 86 kDa antigen (TSTA).
15. gi|20070125: **Protein-Disulfid-Isomerase (PDI);** protein disulfide isomerase (PDI); Prolyl-4-hydroxylase beta; protein disulfide oxidoreductase; thyroid hormone binding protein p55; glutathione insulin transhydrogenase.
16. gi|4557581: **epidermales Fettsäure-bindendes Protein (E-FABP);** Fatty acid binding protein 5 (FABP-5); epidermal fatty acid-binding protein (E-FABP); Psoriasis-associated fatty acid-binding protein (PA-FABP); cutaneous fatty acid-binding protein (C-FABP); keratinocyte lipid binding protein (KLBP); DA11.
17. gi|12707570: **mitochondriale Enoyl-Coenzym A-Hydratase**; Mitochondrial Enoyl Coenzyme A hydratase; Mitochondrial enoyl-CoA hydratase; short-chain enoyl-CoA hydratase, mitochondrial; short-chain enoyl-coenzyme A hydratase (SCEH).
18. gi|16307090: **Nukleophosmin;** Nucleophosmin; nucleolar phosphoprotein B23; Nucleolar protein NO38; numatrin; NPM(1 ).
19. gi|7768772: **HES1 Protein,** homolog zu E. coli und Zebrafisch ES1 Protein, anti-sigma cross-reacting protein homolog 1 alpha precursor, KNP-la/Kpn-1 alpha, GT335, ähnlich zu E. coli SCRP27A und zu Zebrafisch ES1 [Homo sapiens].
20. gi|4506181: **Proteasomen alpha 2-Untereinheit;** proteasome subunit HC3, proteasome component C3; macropain subunit C3; multicatalytic endopeptidase complex subunit C3 [Homo sapiens].
21. gi|4502171: **Adenine-Phosphoribosyltransferase;** AMP pyrophosphorylase; AMP diphosphorylase; transphosphoribosidase.
22. gi|11056044: **anorganische Pyrophosphatase;** cytosolic inorganic pyrophosphatase; inorganic pyrophosphatase 1; pyrophosphate phospho-hydrolase [Homo sapiens].
   Darüber hinaus konnten noch vier weitere Proteine identifiziert werden, die jeweils in Krebsgewebe verglichen mit Kontrollgewebe in bestimmten Patientengruppen (Clusteranalyse) heraufreguliert bzw. herabreguliert sind. Und zwar handelt es sich hierbei um die Proteine Annexin A3, Transgelin, Triosephosphat-Isomerase und Aldolase A. Annexin A3 wird in Krebsgewebe etwa 5-fach heraufreguliert und Transgelin wird etwa 5-fach herabreguliert. Triosephosphat-Isomerase und Aldolase A wird jeweils in Krebsgewebe um etwa 20 % bzw. etwa 10 % heraufreguliert.
   In diesem Zusammenhang wird auf die Fig. 3 verwiesen, die in graphischer Weise die Ergebnisse der Clusteranalyse darstellt. Hieraus ergibt sich die Herauf- bzw. Herabregulation verschiedener Proteine in Krebsgewebe bestimmter Patientengruppen bzw. bestimmter Cluster, die jeweils durch einen Kreis dargestellt sind, im Vergleich mit gesundem Gewebe.
   Die englischsprachigen Synonyme für Annexin A3 und Transgelin lauten folgendermaßen:
23. gi|4826643: **Annexin A3;** Annexin III; lipocortin III; anticoagulant protein III; Placental anticoagulant protein III (PAP III); 35 alpha calcimedin.
24. gi|4507359: **Transgelin;** SM22-alpha smooth muscle protein, 22 kDa actin-binding protein, Smooth muscle 22 protein, Actin-associated protein p27, 25 kDa F-actin-binding protein.

Zusätzlich wurden noch weitere Proteine identifiziert, die im Vergleich von Krebsgewebe mit Kontrollgewebe in bestimmten Patientengruppen abweichende Abundanz zeigten bzw. herauf- bzw. herabreguliert waren. Bei diesen Proteinen handelt es sich um ATP-Synthase, Biliverdin-Reduktase B, Glucose-reguliertes Protein, Prolyl 4-Hydroxylase beta und dnak-artiges molekulares Chaperone. Und zwar ist die ATP-Synthase herabreguliert und die übrigen dieser Proteine sind heraufreguliert.

Interessanterweise weisen viele der identifizierten Proteine eine Beziehung zum Lipidmetabotismus auf. Für Annexin A3 und SAP wurde eine direkte Bindung an Lipide beschrieben. Beide Proteine sind an phagozytotischen Vorgängen beteiligt. Mit FABP-3 und E-FABP wurden zwei Fettsäure-bindende Proteine identifiziert. Die mitochondriale Enoyl-Coenzym A-Hydratase ist an der β-Oxidation von Fettsäuren beteiligt. Die Aktivität von HSP27 wird durch die Aktivität der Protein-Kinase C stimuliert, die selbst durch die Aktivität von Phospholipasen beeinflußt wird, welche Phospholipide umsetzen. Auch HSP90 beeinflußt den Phospholipid-Stoffwechsel, da eine Inhibierung von HSP90 in einem veränderten Phospholipid-Metabolismus resultiert (Chung Y. L. et al., 2003, J. Natl. Cancer Inst. 95: 1624-33). Weiterhin ist PDI vermutlich ebenfalls mit dem Lipid-Metabolismus verknüpft, da PDI als ein multifunktionales Protein wirkt, welches u.a. am Triglycerid-Transfer beteiligt ist (Horiuchi R. and Yamauchi K., 1994, Nippon-Rinsho 52: 890-5). Darüberhinaus hemmen die 14-3-3 Proteine die Aktivität der Protein-Kinase C und enthalten konservierte Sequenzen, die der Pseudosubstrat-Domäne der Protein-Kinase C und dem C-Terminus der Annexine ähneln. Dies deutet alles darauf hin, dass eine funktionelle Beziehung zwischen diesen verschiedenen Proteinen besteht.

Mit Annexin A3 wird Prostatakrebs, insbesondere Prostatakarzinom Diagnostiziert. Wie schon eingangs erwähnt, stellen Prostatakarzinome die häufigsten malignen Tumore bei Männern dar. Nur wenn ein Prostatatumor in einem frühen Stadium nachgewiesen werden kann, kann eine vorbeugende chirurgische Entnahme der Prostata eine erfolgversprechende Therapie sein. Für die fortgeschrittene, nicht mehr auf ein Organ begrenzte Erkrankung ist eine vorbeugende Entnahme der Prostata nicht mehr ausreichend. Für diese zum Teil inoperativen Prostatatumore kann eine Hemmung der männlichen Sexualhormone in Betracht gezogen werden. Eine solche Hemmung, vorzugsweise in Kombination mit einer chirurgischen oder pharmakologischen Kastration, inhibiert zum Teil die Proliferation und Metastasierung des Tumors und erlaubt damit dessen Kontrolle für einen gewissen Zeitraum. Die meisten Prostatatumore entwickeln allerdings mit der Zeit eine gewisse Resistenz gegenüber dieser endokrinologischen Therapie. Andere Therapiemöglichkeiten, wie z. B. die Verwendung von cytotoxischen Agenzien, Gentherapie oder Immunotherapie befinden sich in der klinischen Erprobung, konnten bisher leider noch keinen durchschlagenden Erfolg erzielen. Dies macht es erforderlich, daß ein Prostatakrebs möglichst in einem sehr frühen Stadium erkannt wird, so daß dann noch mit Erfolg chirurgisch behandelt werden kann.

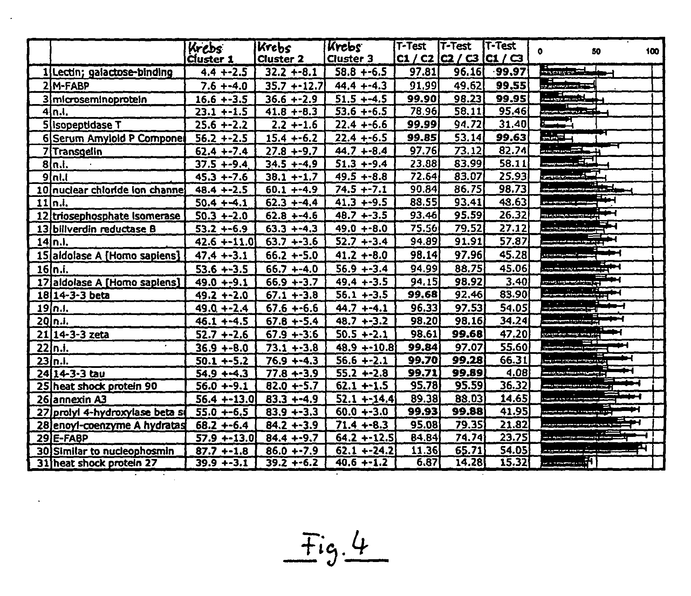

In der erfindungsgemäßen Verwendung von Annexin A3 kann durch die Untersuchung von mehrere Proteine eine bestimmte Unterart von Prostatakrebs, diagnostiziert werden. Die Erfinder konnten zeigen, daß bei einer sogenannten Clusteranalyse bestimmte Proteinmuster eine charakteristische Herauf- bzw. Herabregulation verschiedener Proteine widerspiegeln, welche mit bestimmten Patientengruppen korrelieren. Hierbei weisen die Patienten einer Gruppe jeweils eine bestimmte Unterart von Prostatakrebs, auf. Es wird diesbezüglich auf die Fig. 3 verwiesen, welche in graphischer Weise die Proteinmuster darstellt, welche für die verschiedenen Patientengruppen charakteristisch sind. Fig. 4 zeigt in tabellarischer Form eine Zusammenfassung der Proteinmuster, die die verschiedenen Patientengruppen bzw. Unterarten von Prostatakrebs repräsentieren.

Für eine Diagnose der verschiedenen Unterarten von Prostatakrebs wird eine Kombination verschiedener Proteine in ihrer Abundanz untersucht. Hierbei wird Annexin A3 in Kombination mit mindestens einem der folgenden Proteine für die drei Unterarten (Patientengruppen) von Prostatakrebs analysiert.

Unterart a: heraufreguliertes Transgelin, stark herabreguliertes Galektin, stark herabreguliertes Mikroseminoprotein beta, herabreguliertes Fettsäure-bindendes Protein 3, keine oder geringe Veränderungen von epidermalem Fettsäure-bindenden Protein, keine oder geringe Veränderungen des nukleären Chloridionenkanal-Proteins, keine oder geringe Veränderungen des 14-3-3 Proteins beta, keine oder geringe Veränderungen des 14-3-3 Proteins zeta, keine oder geringe Veränderungen des 14-3-3 Proteins tau, keine oder geringe Veränderungen der Aldolase A, keine oder geringe Veränderungen der Serum-Amyloid P-Komponente, keine oder geringe Veränderungen der Triosephosphat-Isomerase und/oder keine oder geringe Veränderungen von Annexin A3.

Unterart b: stark heraufregulierte Protein-Disulfid-Isomerase, stark heraufreguliertes Hitzeschockprotein 90, stark herabregulierte Ubiquitinlsopeptidase T, heraufreguliertes 14-3-3 Protein beta, heraufreguliertes 14-3-3 Protein zeta, heraufreguliertes 14-3-3 Protein tau, heraufregulierte Aldolase A, heraufregulierte Triosephosphat-Isomerase, heraufreguliertes Annexin A3, herabreguliertes Transgelin, herabreguliertes Galektin, herabreguliertes Mikroseminoprotein beta, herabregulierte Serum-Amyloid P-Komporiente, keine oder geringe Veränderungen des Fettsäure-bindenden Proteins 3 und/oder keine oder geringe Veränderungen des nukleären Chloridionenkanal-Proteins.

Unterart c: stark heraufreguliertes nukleäres Chloridionenkanal-Protein, herunterregulierte Serum-Amyloid P-Komponente, keine oder geringe Veränderungen des Fettsäure-bindenden Proteins 3, keine oder geringe Veränderungen des 14-3-3 Proteins beta, keine oder geringe Veränderungen des 14-3-3 Proteins zeta, keine oder geringe Veränderungen des 14-3-3 Proteins tau, keine oder geringe Veränderungen der Aldolase A, keine oder geringe Veränderungen der Triosephosphat-Isomerase, geringe Veränderungen von Annexin A3, keine oder geringe Veränderungen des epidermalen Fettsäure-bindenden Proteins, keine oder geringe Veränderungen von Mikroseminoprotein beta, keine oder geringe Veränderungen von Galektin und/oder keine oder geringe Veränderungen von Transgelin.

Bei der erfindungsgemäßen Verwendung der beschriebenen Proteine als diagnostische Marker können verschiedene Methoden eingesetzt werden, um die Häufigkeit bzw. Abundanz der Proteine in Krebsgewebe (bzw. in zu untersuchendem Gewebe) im Vergleich mit Kontrollgewebe zu analysieren. Besonders vorteilhaft ist es, wenn die Proteine der zu untersuchenden Probe und der Kontrollprobe gelelektrophoretisch aufgetrennt werden, beispielsweise auf einem üblichen Polyacrylamidgel. Anschließend wird die Abundanz der jeweiligen Proteine in Probe und Kontrolle miteinander verglichen. Wegen der erforderlichen Auflösung sind hierbei vor allem zweidimensionale Gele bevorzugt. Andererseits ist es auch möglich, vor der gelelektrophoretischen Auftrennung eine Vorreinigung vorzunehmen, so daß auch beispielsweise mit einer eindimensionalen Polyacrylamid-Gelelektrophorese eine ausreichende Auftrennung und Analysierbarkeit erreicht wird. Auch andere Methoden zur Auftrennung von Proteinen können mit Vorteil eingesetzt werden, beispielsweise übliche chromatographische Methoden, insbesondere säulenchromatographische Methoden. Besonders vorteilhaft ist es, wenn die zu untersuchende Probe und die Kontrollprobe auf unterschiedliche Weise markiert wird, beispielsweise durch verschiedene Isotope. Hierdurch wird ein Vergleich der zu untersuchenden Probe mit der Kontrolle in Bezug auf die Abundanz der jeweiligen Proteine erleichtert. In einer weiteren bevorzugten Ausführungsform werden die zu analysierenden Proteine massenspektrometrisch untersucht, um eine genaue Identifizierung der Proteine zu ermöglichen. So ist z.B. die "Surface Enhanced Laser Desorption lonisation"-Methode (SELDI-Methode) bei Geweben oder Körperflüssigkeits-Präparaten anwendbar. Aber auch in vivo bildgebende Verfahren, insbesondere Positronen-Emmissions-Tomographie (PET) können mit Vorteil eingesetzt werden.

Weiterhin werden die zu untersuchenden Proteine mit Hilfe von Molekülen qualitativ und quantitativ charakterisiert, die gegen die jeweils zu untersuchenden Proteine, die als diagnostische Marker verwendet werden, gerichtet sind. In besonders vorteilhafter Weise handelt es sich bei den Molekülen um Antikörper, insbesondere um polyklonale und/oder monoklonale Antikörper.
Aber auch alle anderen dem Fachmann in diesem Zusammenhang bekannten Affinitätsreagenzien sollen vom Gegenstand der Erfindung erfasst sein.
Zum qualitativen und vor allem quantitativen Nachweis können übliche Immunoassays eingesetzt werden, wie beispielsweise herkömmliche *enzyme-linked immunosorbent assays* (ELISA). Weiterhin können auch immunohistochemische Verfahren und/oder ProteinChips eingesetzt werden. So ist z.B. auch die SELDI-Methodik anwendbar. Für den Nachweis können beispielsweise Körperflüssigkeiten oder Tumorgewebe untersucht werden. Insbesondere für den Nachweis von Annexin A3, 14-3-3 Protein beta, 14-3-3 Protein tau, 14-3-3 Protein zeta und/oder SAP sind Antikörper geeignet. Beispielsweise färbt der Pan anti 14-3-3 beta/zeta monoklonale Antikörper ( Stressgen Katalognummer KAM-CC012C) Epithel- und Krebszellen sowie einige Lymphozyten im Stroma an. Der Maus monoklonale Antikörper gegen das Protein Serum-Amyloid P-Komponente (SAP) (Stressgen Katalognummer HYB 281-05, Arbeitsverdünnung 1:10) färbt das Stroma aber keine Epithel- oder Krebszellen an.

Die erfindungsgemäß festgestellte charakteristische Veränderung der Abundanz der verschiedenen Proteine gegenüber Kontrollgeweben wirkt sich insbesondere auch auf die Aktivität der jeweiligen Proteine, beispielsweise auf deren enzymatische Aktivität, aus. Daher ist es weiterhin bevorzugt, daß neben der Untersuchung der Abundanz oder als Alternative dazu die Aktivität der jeweiligen Proteine bestimmt wird und mit Kontrollen verglichen wird. Auch dies ist unter der Herauf- bzw. Herabregulation der verschiedenen Proteine zu verstehen. Eine entsprechende Untersuchung kann beispielsweise durch übliche enzymatische Tests erfolgen, die sich für die jeweiligen Proteine dem Fachmann erschließen. Weiterhin können beispielsweise im Fall der Fettsäure-bindenden Proteine entsprechende Bindungsassays oder vergleichbares durchgeführt werden, um so Erkenntnisse über die Aktivität bzw. die Herauf- oder Herabregulation dieser Proteine zu gewinnen. Ähnliches gilt auch für die anderen Proteine. Beispielsweise können bestimmte Kanalaktivitäten gemessen werden, um Aussagen über das nukleäre Chloridionenkanal-Protein (CLIC-1) machen zu können. Dies kann für die Verwendung der verschiedenen Proteine als diagnostische Marker bzw. den im folgenden beschriebenen erfindungsgemäßen Diagnosekit ausgenutzt werden. Weiterhin kann eine derartige Messung der Aktivität der jeweiligen Proteine durchgeführt werden, um die Wirkung eines Wirkstoffs zu testen, der erfindungsgemäß zur Herstellung eines Medikaments zur Behandlung von Krebs verwendet wird, wie nachfolgend beschrieben wird.

In einer weiteren bevorzugten Ausführungsform der erfindungsgemäßen Verwendung werden für eine Untersuchung des mindestens einen Proteins Exosomen aus beispielsweise Patientenmaterial isoliert und bezüglich des oder der Proteine analysiert. Insbesondere wird das Proteinmuster eines oder mehrerer Proteine innerhalb der Exosomen untersucht, so daß hiermit die diagnostisch relevante Herauf- und/oder Herabregulation einzelner oder mehrerer Proteine festgestellt werden kann. Eine geeignete Isolierung der Exosomen aus beispielsweise Patientenmaterial kann mit üblichen Methoden erfolgen, die dem Fachmann geläufig sind.

Die zu untersuchenden Proben und die Kontrollproben stammen im allgemeinen aus dem Körper eines Patienten, wobei beispielsweise Gewebeproben oder auch Proben aus Körperflüssigkeiten, wie beispielsweise Blut, Serum, Lymphflüssigkeit oder Urin, entnommen und in der für einen Fachmann geläufigen Weise aufbereitet werden. Besonders vorteilhaft ist es, wenn potentiell bösartiges Gewebe, also das typischerweise zu untersuchende Gewebe, und Kontrollgewebe, also gutartiges Gewebe, aus dem selben Patienten entnommen werden und direkt miteinander verglichen werden. Andererseits ist es auch möglich, die Abundanz der jeweiligen Proteine mit anderen Standards zu vergleichen, die beispielsweise aus einer Vielzahl von unabhängigen Kontrollproben statistisch ermittelt werden. Im Fall von Prostatakrebs wird vorteilhafterweise benignes und potentiell malignes Prostatagewebe aus einem Patienten entnommen, der z.B. einer Prostatektomie oder Biopsie unterzogen wird. Bei dem Kontrollgewebe kann es sich beispielsweise um gutartiges Prostatahyperplasia-Gewebe handeln.

### Beispiele

Zur Identifizierung von erfindungsgemäß relevanten Proteinen wurden die Gewebeproben von zwei Patientengruppen (Gruppe A: 23 Patienten und Gruppe B: 33 Patienten) untersucht. Krebsgewebe und Kontrollgewebe wurde jeweils aufbereitet und einer zweidimensionalen Polyacrylamid-Gelelektrophorese (2D-PAGE) unterzogen. Die isoelektrische Fokussierung erfolgte bei pH 4-7 und pH 6-11. Die gelelektrophoretischen Ergebnisse von zwei Patienten aus der Gruppe A waren für eine weitere Analyse ungeeignet. Bei weiteren zwei Patienten ergaben die Ergebnisse bei pH 6-11 unbefriedigende Resultate. Insgesamt konnten also die Ergebnisse von 21 Patienten im pH-Bereich von 4-7 und die Ergebnisse von 19 Patienten im pH-Bereich von 6-11 ausgewertet werden. Aus der Gruppe B waren die Ergebnisse von 2 Patienten bei pH 4-7 für eine weitere Analyse ungeeignet. Insgesamt konnten daher die Ergebnisse von 31 Patienten im pH-Bereich von 4-7 ausgewertet werden.

Die beiden verschiedenen Proben jedes Patienten wurden mit jeweils unterschiedlichen Isotopen markiert, gemischt und auf einem einzigen zweidimensionalen Polyacrylamidgel elektrophoretisch aufgetrennt. Die Signale jedes Isotops wurden anschließend getrennt voneinander detektiert, so daß die Proteinmuster der beiden Gewebeproben unmittelbar miteinander verglichen werden konnten (Proteo-Tope-Technologie).

Zur letztendlichen Identifizierung der Proteine wurden analytische Mengen (< 1 µg) der radiomarkierten Proteine mit präparativen Mengen (> 200 µg) der Proteine der selben Probe zusammen in präparativen Anreicherungsgelen aufgetrennt. Die interessanten Proteinspots wurden aus silbergefärbten präparativen Gelen ausgeschnitten, tryptisch verdaut und durch *matrix assisted laser desorption ionization time of flight mass spectrometry* (MALD1-TOF MS) identifiziert (Bruker BiFlex oder Ultra-Flex). Teilweise wurde eine *electrospray ionization ion trap mass spectrometry* (ESI-MS) durchgeführt (Bruker Esquire).

Auf diese Weise wurden verschiedene Proteine eindeutig identifiziert, die in übereinstimmender Weise eine erhöhte oder eine erniedrigte Abundanz in Krebsgewebe im Vergleich mit Kontrollgewebe aufwiesen.

Bei den Analysen wurden zum Teil bestimmte Patientengruppen gebildet, innerhalb derer die Abundanzen der verschiedenen Proteine untersucht wurden. Bei dieser sogenannten Cluster-Analyse (Clustan Graphics 6.4) wurden drei Gruppen von Patienten aus Gruppe A und zwei Gruppen von Patienten aus Gruppe B bestimmt, die jeweils charakteristische Proteinexpressions-/Abundanzmuster zeigten. Mit Hilfe dieser Vorgehensweise wurden die Proteine Annexin A3, Transgelin, Triosephosphat-Isomerase und Aldolase A identifiziert, die für bestimmte Patientengruppen charakteristische Abundanzen zeigten.

### Gewebeproben

Gesundes Prostatagewebe und malignes Prostatagewebe wurde von Patienten, die zuvor einer Prostatektomie unterzogen worden waren, erhalten. Die Patienten wurden mit Hilfe des PSA- (prostata specific antigen) Screening untersucht, und die Tumore wurden mit Ultraschall bestätigt. Eine Einwilligung jedes Patienten wurde vor Durchführung der Operation eingeholt.

Sofort nach Entnahme der Prostata wurde diese in eine sterile Box überführt und dort gekühlt. Es wurden 0,5 - 1 cm dicke Gewebeschnitte angefertigt, die in eine linke und eine rechte Hälfte unterteilt wurden. Diese wurden in eine *freezing matrix* eingebettet und schockgefroren. Der Rest der Prostata wurde in Formalin fixiert und gemäß üblichen Standardverfahren weiterbehandelt. Zur Herstellung von Gewebeproben wurden dünne Sektionen von beiden Seiten der Prostata entnommen und mit Hämatoxilin-Eosin angefärbt. Diese Schnitte wurden bis zur Verwendung bei -80 °C gelagert. Kontrollgewebeproben wurden aus nichttumorbefallenen Regionen entnommen und einer identischen Behandlung unterzogen.

### Probenaufbereitung

Die Proteine wurden in 100 µl kochendem 2 % SDS, 0,1 M Tris, pH 8,8 lysiert und die Proteinkonzentration anhand der *Bicinchoninic acid-*Methode (BCA) bestimmt.

Die lodierung mit ¹²⁵I bzw. ¹³¹I, die zweidimensionale Polyacrylamid-Gelelektrophorese sowie die Datenanalyse wurde nach herkömmlichen Verfahren durchgeführt (Cahill et al., 2003, Rapid Communications in Mass Spectrometry 17: 1283-1290). Radioaktives lod stammte von Amersham Bioscience (Freiburg). Die Proteiniodierungsreaktionen entweder mit ¹²⁵I oder ¹³¹I wurden jeweils separat mit identischen lodkonzentrationen durchgeführt.

### Polvacrylamid-Gelelektrophorese

Für den Auftrag auf die Polyacrylamidgele wurden identische Proteinmengen der markierten Proben (Krebsgewebe und Kontrollgewebe) miteinander gemischt. Für die isoelektrische Fokussierung (IEF) über die pH-Bereiche 4-7 und 6-11 wurden die Proben in einem üblichen Probenpuffer auf 18 cm immobilisierte pH-Gradienten (IPG)-Streifen (Amersham Bioscience) geladen. IEF als erste Dimension der 2D-PAGE-Proteinauftrennung wurde auf einer Multiphor-Apparatur (Amersham Bioscience) durchgeführt. Die zweite Dimension (SDS-PAGE) wurde auf einer ISO-DALT-Apparatur (Höfer) durchgeführt. Die Gele wurden getrocknet, auf einen 80 µm Plastikfilm laminiert, und anschließend wurde die radioaktive Messung der Signale von beiden Radioisotopen vorgenommen.

Durch die gewählte Analyse der verschiedenen Radioisotope konnte eine quantitative, mehrfarbige differentielle Darstellung der Proteine der verschiedenen Proben erreicht werden. Daher konnte ein direkter Vergleich der integrierten Proteinspotintensitäten der beiden Proben, die auf einem Gel aufgetrennt waren, für die weitere Analyse verwendet werden. Die Analyse auf einem Gel hat den Vorteil, daß Variationen zwischen zwei oder mehr Gelen nicht ins Gewicht fallen. Die größte potentielle Fehlerquelle ist eine unterschiedliche stöchiometrische Markierung mit dem einen oder dem anderen Isotop. Dies wurde durch die Herstellung von Gelen mit jeweils umgekehrten Markierungen ausgeschlossen. Das heißt also, daß die Kontrollprobe und die Krebsgewebeprobe jeweils mit dem einen und auch mit dem anderen Isotop markiert wurden und jeweils invers miteinander verglichen wurden. Da die Proteinmuster der jeweils inversen Markierungsprozeduren übereinstimmten, waren die qualitativen Kriterien erfüllt. Mit Hilfe von computergestützten Vorgehensweisen wurden die verschiedenen Isotopsignale jeweils in einer anderen Farbe (blau bzw. orange) sichtbar gemacht, so daß konsistente Unterschiede in der Abundanz zwischen den Proben jeweils in der einen bzw. anderen Farbe je nach gewählter Isotopenmarkierung erschien. Näheres zu dieser Vorgehensweise ergibt sich aus Cahill et al., 2003, Rapid Communications in Mass Spectrometry 17: 1283-1290.

### Bildanalyse

Die differentielle Proteinabundanzanalyse basiert auf der zuverlässigen differentiellen Quantifizierung der Proteinspots auf den beschriebenen Polyacrylamidgelen. Für eine quantitative Bildanalyse wurde die Phoretix 2D Advanced software (Nonlinear Dynamics) angewendet, wobei eigene Anpassungen vorgenommen wurden.

### Proteinidentifizieruna durch Massenspektrometrie

Es wurden im Prinzip zwei massenspektrometrische Methoden eingesetzt. Zum einen die sehr schnelle und zuverlässige Identifizierung von hoch abundanten Proteinen durch *peptide mass fingerprinting* mit MALDI-TOF MS. Die Identifizierung von sehr niedrig abundanten Proteinen wurde mit der zeitaufwendigeren LC-ESI-IonTrap-MS/MS oder MALDI-TOF-TOF Methode durchgeführt. Zusammengefaßt wurden Gelstücke der ausgewählten Proteinspots ausgeschnitten und die Proteine in diesen Gelstücken mit Hilfe von Trypsin verdaut. Die resultierende Lösung wurde zunächst mit der *peptide mass fingerprint*-Prozedur basierend auf MALDI-TOF-MS analysiert. Für die Proteinspots, für die auf diese Weise keine eindeutige Identifikation möglich war, wurde die langsamere Fragmentionenanalyse, basierend auf MALDI-TOF-TOF oder LC-ESI-IonTrap-MS/MS zusätzlich durchgeführt. Eine genaue Beschreibung dieser Methoden ist Vogt et al., 2003, Rapid Communication in Mass Spectrometry 17: 1273-1282 und Vogt et al., 2003, Molecular Cellular Proteomics 2: 795 zu entnehmen.

### Identifizierung der Proteine

Zur Identifizierung der Proteine wurden die Peptidmassen, die durch die Massenspektrometrie gewonnen wurden, gegenüber der NCBI-Proteindatenbank ausgewertet. Dies wurde mit Hilfe des Programms MASCOT Version 1.9 (Matrix Science, London, UK) durchgeführt.

### Quantitative Bildanalyse

Quantitative Analysen wurden mit Hilfe der digitalen Daten durchgeführt, die mit einem Photomultiplier eines Radio-Imagers für jeden Pixel der Bildmatrix aufgezeichnet wurden. Die Proteinspotgrenzen wurden mit Hilfe der Software Phoretix 2D Advanced (Nonlinear Dynamics) definiert und die Pixelwerte innerhalb der Spotregion nach Subtraktion eines geeigneten Hintergrundsignals integriert. Basierend auf den kompletten Daten, die generiert wurden, wurde eine detaillierte Quantifizierung der detektierten Proteinspots durchgeführt. Die Tabelle 1 enthält eine Zusammenfassung dieser Ergebnisse.

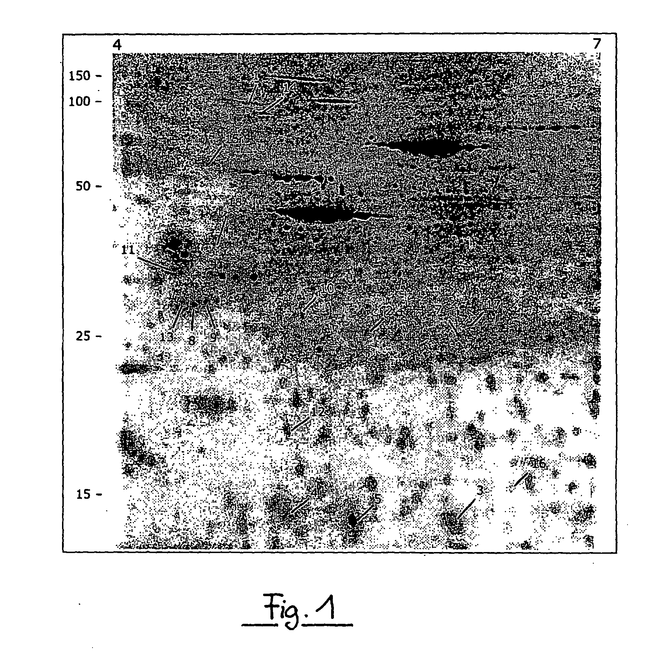

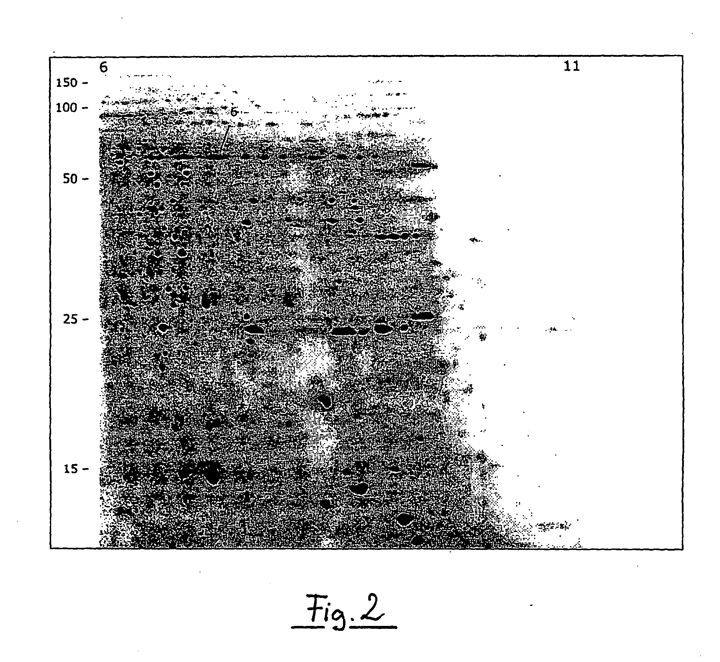

Die Figuren 1 und 2 zeigen jeweils die Positionen der ausgewählten Proteinspots, einmal bei isoelektrischer Fokussierung bei pH 4-7 (Fig. 1) und im anderen Fall bei pH 6-11 (Fig. 2).

In den Abbildungen zeigen:
- Fig. 1:: Darstellung eines zweidimensionalen Polyacrylamidgels mit aufgetrennten Proteinen eines Patienten aus der Gruppe A. Die isoelektrische Fokussierung erfolgte bei pH 4-7. Die mit Zahlen markierten Proteinspots zeigen diejenigen Proteine, die abweichende Abundanz in Krebsgewebe und Kontrollgewebe zeigen. Die Numerierung entspricht der Tabelle 1.
- Fig. 2:: Darstellung eines zweidimensionalen Polyacrylamidgels mit aufgetrennten Proteinen eines Patienten aus der Gruppe A. Die isoelektrische Fokussierung erfolgte bei pH 6-11. Die mit Zahlen markierten Proteinspots zeigen diejenigen Proteine, die abweichende Abundanz in Krebsgewebe und Kontrollgewebe zeigen. Die Numerierung entspricht der Tabelle 1.
- Fig. 3:: Graphische Darstellung der Proteinmuster, die für bestimmte Patientengruppen bzw. Unterarten von Prostatakrebs charakteristisch sind. Ergebnisse mit T-Testwahrscheinlichkeiten von p < 0,01 sind schwarz dargestellt, Ergebnisse mit T-Testwahrscheinlichkeiten von 0,01 < p < 0,1 sind grau dargestellt. Proteine, die in ihrer Abundanz innerhalb der verschiedenen Cluster variieren, sind eingerahmt.
- Fig. 4:: Tabellarische Darstellung der verschiedenen Proteinlevel der Patientengruppen 1 bis 3 aus der Gruppe A im Vergleich von gutartigem (gesundem) und bösartig entartetem Gewebe. Die Angaben beziehen sich auf Prozent der Proteinspotgröße von Krebsgewebe mit Standardfehler im Verhältnis zum gesamten Proteinspotvolumen (gutartig + bösartig). Das T-Testergebnis ist als die Wahrscheinlichkeit dargestellt, daß die Verteilung der Spotfraktionen aus zwei gegebenen Gruppen signifikant unterschiedlich ist. T-Testergebnisse von wenigstens 99 % sind fettgedruckt. Ergebnisse von unterhalb 95 % sind hellgrau dargestellt.
- Fig. 5:: Tabellarische Liste der Proteinspots mit signifikanter differentieller Abundanz der Patienten aus der Gruppe A im Vergleich von gutartigem (gesundem) und bösartigem Gewebe, basierend auf der Zweifarben-ProteoTope-Analyse. "No. Obs." gibt die Anzahl der Patienten wieder, bei welchen der Spot beobachtet werden konnte. Die Spotfraktion für gutartiges (benigne Faktion) und bösartiges (Krebs-Fraktion) mit Standardfehler ist in Prozent des gesamten Spotvolumens (gutartig + bösartig) angegeben. Das T-Testergebnis ist als Wahrscheinlichkeit angegeben, daß die Verteilung der Spotfraktionen für gutartiges Gewebe von der Verteilung, die in Krebsgewebe gefunden wurde, abweicht, wobei alle Patienten berücksichtigt wurden. Die Spots wurden unter der Voraussetzung ausgewählt, daß die T-Testwahrscheinlichkeit wenigstens 99 % beträgt.

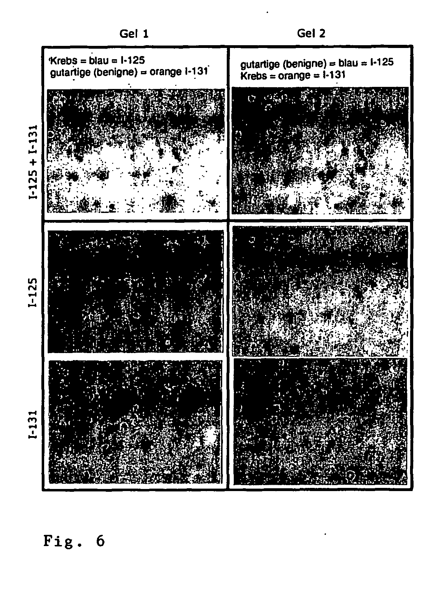

- Fig. 6:: Darstellung eines zweidimensionalen Polyacrylamidgels mit aufgetrennten Proteine des Patienten 14 aus der Gruppe B. Sowohl die Kontrollprobe als auch die Krebsgewebeprobe werden mit ¹³¹I und ¹²⁵I markiert und invers miteinander verglichen. Die verschiedenen lsotopsignale sind jeweils in einer anderen Farbe (Blau bzw. Orange) sichtbar gemacht, so daß konsistente Unterschiede in der Abundanz von Proteinen zwischen den Proben jeweils in der einen bzw. anderen Farbe, je nach gewählte Isotopenmarkierung, erscheinen.

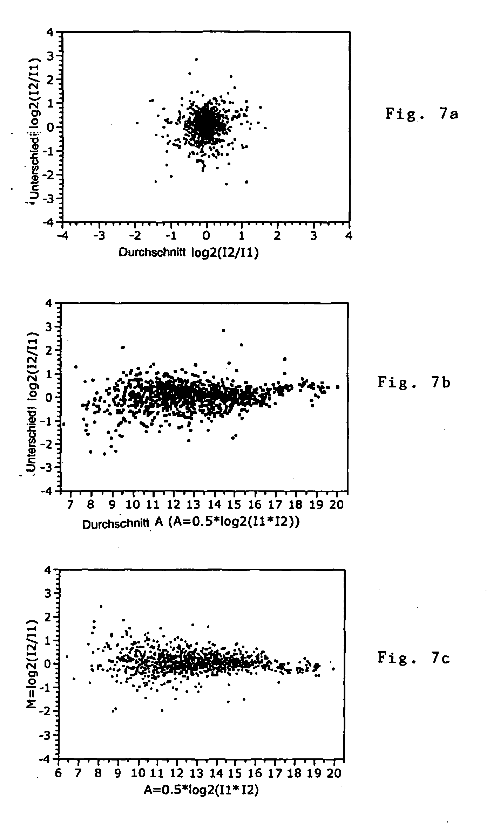

- Fig. 7:: Graphische Darstellungen, die die Genauigkeit und die statistische Bedeutung der Proteo-Tope Messungen am Beispiel der Gruppe B zeigen:
a: Bland und Altman Plot, der das Verhältnis zwischen dem Unterschied in dem differenziellen Abundanzverhältnis M und ihrem arithmetischen Mittel für ein mit ¹²⁵I und ¹³¹I markiertes Gel wiedergibt,
b: Plot, der das Verhältnis zwischen dem Unterschied in dem differenziellen Abundanzverhältnis M und dem arithmetischen Mittel der Intensität A für ein mit ¹²⁵I und ¹³¹I markiertes Gel wiedergibt,
c: MA-Plot, der das Verhältnis zwischen dem differenziellen Abundanzverhältnis M und der Intensität A für ein mit ¹²⁵I und ¹³¹I markiertes Gel,
wobei M = log2 · (I2/I1) und A = 0,5 · log2 · (I1 · I2) ist (I = gemessene Intensität).

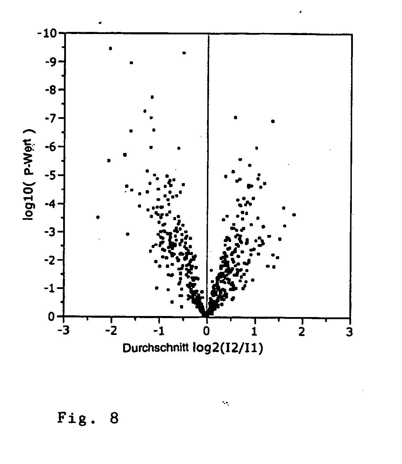

- Fig. 8:: Volcano Plot, der den Unterschied zwischen den Durchschnittsintensitäten der nachgewiesenen invers markierten Proteine aus karzinomatösem und gutartigen Gewebe zeigt.

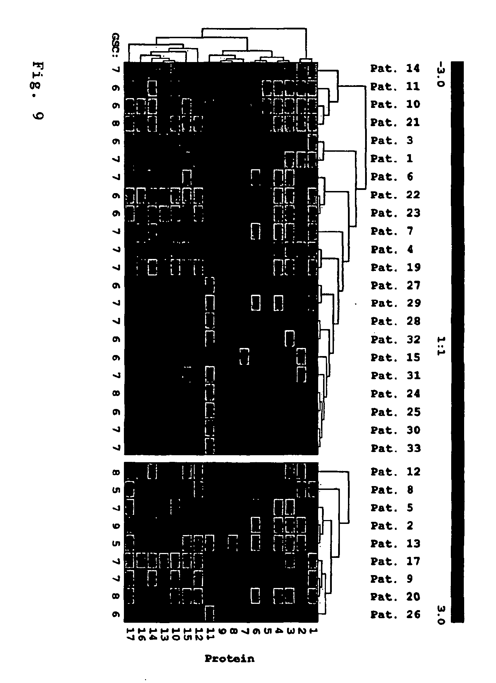

- Fig. 9:: Grafische Darstellung der Pavlidis Templat Matching Analyse, die unter den Krebspatienten aus Gruppe B zwei Untergruppen von Proteinabundanzverhältnismustern ergibt. Eine Gruppe besteht aus 22 Patienten, die andere Gruppe, die sich wesentlich davon unterscheidet, aus 9 Patienten. Die Proteinnummern entsprechen den Nummern in der Tabelle von Fig. 10. Innerhalb der Untergruppe aus 22 Patienten unterscheidet sich die relative Abundanz von Annexin A3 (Protein 14) deutlich. Das Protein ist bei den Patienten 14, 11, 10, 21, 3, 1, 6, 22, 23, 7, 4, 19 und 27 deutlich abundanter in malignem Prostatagewebe als bei den Patienten 29, 28, 32, 15, 31, 24, 25, 30 und 33.
- Fig. 10:: Tabellarische Darstellung der Proteinspots aus der differenzellen Analyse von allen 31 Patienten (Gruppe B), der Gruppe mit 22 und der Gruppe mit 9 Patienten (erhalten durch die Pavlidis Templat Matching Analyse). Die *Accession Number* entspricht der jeweiligen Nummmer aus der NCBI-Datenbank. Unter "scores" sind die mit Hilfe der MASCOT-Technik ermittelten Treffer zu verstehen. Die Angabe zum PMF-Score bezieht sich auf den Mowse-Score, der vom MASCOT-Server verwendet wird, wobei im allgemeinen ein PMF-Score, der höher als 65 ist, eine signifikante Identifizierung repräsentiert. Die Identiät der mit einem Sternchen versehenen Proteine wurde durch LC/MS/MS bestimmt. Die durchschnittliche Spotfraktion von karzinomatösem Gewebe ist mit Standardfehler in Prozent des gesamten Spotvolumens (gutartig + bösartig) angegeben. Der P-Wert für dieses Model ist ebenfalls angegeben. Die Balkon in der tabellarische Darstellung zeigen die durchschnittliche Abundanz in % eines jeden Proteins in den benignen (dunkel Blau) und karzinomatösen (leicht Orange) Proben in den aufgeführten Patientengruppen.

## Patentansprüche

1. Verwendung des Proteins Annexin A3 als diagnostischer Marker für Prostatakrebs, **dadurch gekennzeichnet, dass** es sich bei dem Prostatakrebs um bestimmte Unterarten von Prostatakrebs handelt, wobei die Unterarten von Prostatakrebs ausgewählt sind aus:
Unterart a, **gekennzeichnet durch** heraufreguliertes Transgelin, stark herabreguliertes Galektin, stark herabreguliertes Mikroseminoprotein beta, herabreguliertes Fettsäure-bindendes Protein 3, keine oder geringe Veränderungen von epidermalem Fettsäure-bindenden Protein, keine oder geringe Veränderungen des nukleären Chloridionenkanal-Proteins, keine oder geringe Veränderungen des 14-3-3 Proteins beta, keine oder geringe Veränderungen des 14-3-3 Proteins zeta, keine oder geringe Veränderungen des 14-3-3 Proteins tau, keine oder geringe Veränderungen der Aldolase A, keine oder geringe Veränderungen der Serum-Amyloid P-Komponente, keine oder geringe Veränderungen der Triosephosphat-Isomerase und/oder keine oder geringe Veränderungen von Annexin A3,
Unterart b, **gekennzeichnet durch** stark heraufregulierte Protein-Disulfid-Isomerase, stark heraufreguliertes Hitzeschockprotein 90, stark herabregulierte Ubiquitin-Isopeptidase T, heraufreguliertes 14-3-3 Protein beta, heraufreguliertes 14-3-3 Protein zeta, heraufreguliertes 14-3-3 Protein tau, heraufregulierte Aldolase A, heraufregulierte Triosephosphat-Isomerase, heraufreguliertes Annexin A3, herabreguliertes Transgelin, herabreguliertes Galektin, herabreguliertes Mikroseminoprotein beta, herabregulierte Serum-Amyloid P-Komponente, keine oder geringe Veränderungen des Fettsäure-bindenden Proteins 3 und/oder keine oder geringe Veränderungen des nukleären Chloridionenkanal-Proteins, oder
Unterart c, **gekennzeichnet durch** stark heraufreguliertes nukleäres Chloridionenkanal-Protein, herunterregulierte Serum-Amyloid P-Komponente, keine oder geringe Veränderungen des Fettsäure-bindenden Proteins 3, keine oder geringe Veränderungen des 14-3-3 Proteins beta, keine oder geringe Veränderungen des 14-3-3 Proteins zeta, keine oder geringe Veränderungen des 14-3-3 Proteins tau, keine oder geringe Veränderungen der Aldolase A, keine oder geringe Veränderungen der Triosephosphat-Isomerase, geringe Veränderungen von Annexin A3, keine oder geringe Veränderungen des epidermalen Fettsäure-bindenden Proteins, keine oder geringe Veränderungen von Mikroseminoprotein beta, keine oder geringe Veränderungen von Galektin und/oder keine oder geringe Veränderungen von Transgelin.

2. Verwendung nach Anspruch 1, **dadurch gekennzeichnet, dass** eine Heraufregulation des Annexin A3 im Vergleich mit Kontrollen untersucht wird.

3. Verwendung nach einem der vorhergehenden Ansprüche, **dadurch gekennzeichnet, dass** eine Heraufregulation von Annexin A3 in Kombination mit einer Herabregulation von Annexin A1, Annexin A2 und/oderAnnexin A5 untersucht wird.

## Claims

1. Use of the protein annexin A3 as a diagnostic marker for prostate cancer, **characterized in that** the prostate cancer is certain subtypes of prostate cancer, where the subtypes of prostate cancer are selected from:
subtype a, **characterized by** up-regulated transgelin, strongly down-regulated galectin, strongly down-regulated microseminoprotein beta, down-regulated fatty acid-binding protein 3, no or slight changes of epidermal fatty acid-binding protein, no or slight changes of the nuclear chloride ion channel protein, no or slight changes of the 14-3-3 protein beta, no or slight changes of the 14-3-3 protein zeta, no or slight changes of the 14-3-3 protein tau, no or slight changes of the aldolase A, no or slight changes of the serum amyloid P component, no or slight changes of the triose phosphate isomerase and/or no or slight changes of annexin A3,
subtype b, **characterized by** strongly up-regulated protein disulfide isomerase, strongly up-regulated heat shock protein 90, strongly down-regulated ubiquitin isopeptidase T, up-regulated 14-3-3 protein beta, up-regulated 14-3-3 protein zeta, up-regulated 14-3-3 protein tau, up-regulated aldolase A, up-regulated triosephosphate isomerase, up-regulated annexin A3, down-regulated transgelin, down-regulated galectin, down-regulated microseminoprotein beta, down-regulated serum amyloid P component, no or slight changes of the fatty acid-binding protein 3 and/or no or slight changes of the nuclear chloride ion channel protein or
subtype c, **characterized by** strongly up-regulated nuclear chloride ion channel protein, down-regulated serum amyloid P component, no or slight changes of the fatty acid-binding protein 3, no or slight changes of the 14-3-3 protein beta, no or slight changes of the 14-3-3 protein zeta, no or slight changes of the 14-3-3 protein tau, no or slight changes of aldolase A, no or slight changes of triose phosphate isomerase, slight changes of annexin A3, no or slight changes of the epidermal fatty acid-binding protein, no or slight changes of the microseminoprotein beta, no or slight changes of galectin and/or no or slight changes of transgelin.

2. Use according to claim 1, **characterized in that** an up-regulation of annexin A3 in comparison with controls is investigated.

3. Use according to one of the previous claims, **characterized in that** an up-regulation of annexin A3 in combination with a down-regulation of annexin A1, annexin A2 and/or annexin A5 is investigated.

## Revendications

1. Utilisation de la protéine annexine 3 en tant que marqueur diagnostique du cancer de la prostate, **caractérisée en ce que** le cancer de la prostate est une sous-espèce déterminée de cancer de la prostate, les sous-espèces de cancer de la prostate étant choisies parmi les suivantes :
sous-espèce a, **caractérisée par** la transgéline à régulation positive, la galectine à régulation fortement négative, la microséminoprotéine bêta à régulation fortement négative, la protéine 3 se liant aux acides gras à régulation négative, l'absence de modifications ou modifications réduites de la protéine épidermique se liant aux acides gras, l'absence de modifications ou modifications réduites de la protéine nucléaire du canal d'ions chlorure, l'absence de modifications ou modifications réduites de la protéine 14-3-3 bêta, l'absence de modifications ou modifications réduites de la protéine 14-3-3 zêta, l'absence de modifications ou modifications réduites de la protéine 14-3-3 tau, l'absence de modifications ou modifications réduites de l'aldolase A, l'absence de modifications ou modifications réduites du composant sérique amyloïde P, l'absence de modifications ou modifications réduites de la triosephosphate isomérase et/ou l'absence de modifications ou modifications réduites de l'annexine A3,
sous-espèce b, **caractérisée par** la protéine-disulfure isomérase à régulation fortement positive, la protéine de choc thermique 90 à régulation fortement positive, l'ubiquitine-isopeptidase T à régulation fortement négative, la protéine 14-3-3 bêta à régulation positive, la protéine 14-3-3 zêta à régulation positive, la protéine 14-3-3 tau à régulation positive, l'aldolase A à régulation positive, la triose-phosphate-isomérase à régulation positive, l'annexine A3 à régulation positive, la transgéline à régulation négative, la galectine à régulation négative, la microséminoprotéine à régulation négative, le composant sérique amyloïde P à régulation négative, l'absence de modifications ou modifications réduites de la protéine 3 se liant aux acides gras et/ou l'absence de modifications ou modifications réduites de la protéine nucléaire du canal d'ions chlorure, ou
sous-espèce c, **caractérisée par** la protéine nucléaire du canal d'ions chlorure à régulation positive, le composant sérique amyloïde P à régulation négative, l'absence de modifications ou modifications réduites de la protéine 3 se liant aux acides gras, l'absence de modifications ou modifications réduites de la protéine 14-3-3 bêta, l'absence de modifications ou modifications réduites de la protéine 14-3-3 zêta, l'absence de modifications ou modifications réduites de la protéine 14-3-3 tau, l'absence de modifications ou modifications réduites de l'aldolase A, l'absence de modifications ou modifications réduites de la triose-phosphate-isomérase, modifications réduites de l'annexine A3, l'absence de modifications ou modifications réduites de la protéine épidermique se liant aux acides gras, l'absence de modifications ou modifications réduites de la microséminoprotéine bêta, l'absence de modifications ou modifications réduites de la galectine et/ou l'absence de modifications ou modifications réduites de la transgeline.

2. Utilisation selon la revendication 1, **caractérisée en ce qu'**une régulation positive de l'annexine A3 est analysée en comparaison avec des témoins.

3. Utilisation selon l'une des revendications précédentes, **caractérisée en ce qu'**une régulation positive de l'annexine A3 est analysée en combinaison avec une régulation négative de l'annexine A1, annexine A2 et/ou annexine A5.
